# FASCICULE DE BREVET EUROPEEN

(11) **EP 1 768 541 B1**
(45) Date de publication et mention de la délivrance du brevet: **26.08.2015**
(21) Numéro de dépôt: 05756916.2
(22) Date de dépôt: 20.06.2005
(51) Int. Cl.: A61B 1/018, A61B 1/00

(54) **SUPPORT D' INSTRUMENTS COMPRENANT UNE BAGUE ET MONTABLE SUR UN ENDOSCOPE**
AUF EINEM ENDOSKOP MONTIERBARER WERKZEUGHALTER MIT RING
TOOLHOLDER MOUNTABLE ON AN ENDOSCOPE AND COMPRISING A RING

(30) Priorité: 18.06.2004 EP 04447144
(43) Date de publication de la demande: 04.04.2007
(73) Titulaire: Université Libre de Bruxelles, 1050 Bruxelles (BE)
(72) Inventeur: DEVIERE, Jacques, 1470 GENAPPE (BE); CAUCHE, Nicolas, 1040 BRUXELLES (BE); DELCHAMBRE, Alain, 1170 BRUXELLES (BE); EVRARD, Sylvie, 1190 BRUXELLES (BE)
(74) Mandataire: Van Malderen, Joëlle
(86) Numéro de dépôt international: PCT/BE2005/000098
(87) Numéro de publication internationale: WO 2005/122862

(56) Documents cités:
- US-A1- 2003 114 732
- US-B1- 6 179 776
- US-B1- 6 352 503
- PATENT ABSTRACTS OF JAPAN vol. 2003, no. 12, 5 décembre 2003 (2003-12-05) -& JP 2003 245241 A (FUJI PHOTO OPTICAL CO LTD), 2 septembre 2003 (2003-09-02)

## Description

### Objet de l'invention

La présente invention se rapporte à un dispositif utilisable en endoscopie.

La présente invention concerne plus particulièrement un support endoscopique encore appelé ci-après dispositif ou table endoscopique et destiné à permettre le positionnement *in situ* d'un endoscope et d'instruments chirurgicaux associés de façon précise.

Le présent dispositif peut néanmoins s'adapter aussi à des systèmes de vision autres qu'un endoscope et connus de l'homme du métier.

### Etat de la technique

L'endoscopie thérapeutique s'est considérablement développée au cours de ces vingt dernières années. Pratiquée par voie perorale ou transanale, l'endoscopie thérapeutique permet, au moyen d'un instrument appelé « endoscope », un accès direct à l'intérieur du tube digestif sans nécessiter l'ouverture de la paroi abdominale ni le passage par la cavité péritonéale, ce qui augmente le confort et diminue la morbidité pour le patient.

Plus précisément, un endoscope se présente classiquement sous la forme d'un tube souple présentant un canal optique et un ou plusieurs canaux opérateurs permettant d'introduire un ou plusieurs instruments chirurgicaux en vue de pratiquer une manipulation chirurgicale *in situ,* c'est-à-dire au niveau de la cible (cavité) à traiter dans le corps du patient.

Cependant, une telle disposition des canaux opérateurs, parallèlement à l'endoscope, limite considérablement les mouvements dans l'espace accessibles aux instruments chirurgicaux l'un par rapport à l'autre et par rapport à l'endoscope, et il conviendrait de disposer d'une solution efficace à ce problème.

Le document US-B1-6,352,503 a proposé un appareil d'endoscopie chirurgicale comprenant un endoscope pourvu d'une zone d'insertion à l'intérieur de laquelle est inséré un dispositif d'observation, et au moins deux bras d'insertion dans lesquels un instrument chirurgical est inséré, ledit appareil ayant la particularité de comprendre en outre un dispositif de positionnement, pour faire varier l'une ou l'autre des distances entre la zone d'insertion de l'endoscope, le premier bras d'insertion et le second bras d'insertion selon une direction essentiellement orthogonale à l'axe de la zone d'insertion de l'endoscope. Ce dispositif de positionnement se présente de préférence sous la forme d'un ballon gonflable ou également d'un « panier » extensible.

Néanmoins, dans cette solution, les mouvements dans l'espace des instruments chirurgicaux sont encore relativement restreints.

Le document US-B1-6-179-776 décrit un dispositif apte à être couplé à un endoscope et se présentant sous la forme d'un étui flexible qui peut être enfilé sur l'endoscope. Cet étui a une longueur telle qu'il enveloppe l'endoscope sur essentiellement toute sa hauteur. Le dispositif est pourvu de lumens qui s'étendent le long des parois de l'étui et à l'extrémité distale desquels peuvent être fixés des instruments chirurgicaux. L'assemblage du dispositif à l'endoscope est réalisé préalablement à l'insertion du dispositif dans le corps du patient. Une fois que le dispositif assemblé à l'endoscope a atteint sa cible in *situ,* sous l'action de moyens de contrôle, l'extrémité distale des lumens est capable de subir des déflections dont l'amplitude est réglée, permettant ainsi un positionnement et une orientation des instruments chirurgicaux par rapport au site à traiter.

### Buts de l'invention

La présente invention vise à fournir un dispsoitif comprenant un dispositif de positionnement et/ou d'orientation d'un endoscope et d'instruments chirurgicaux couplés audit endoscope qui permettrait de positionner et/ou d'orienter précisément dans l'espace lesdits instruments chirurgicaux l'un par rapport à l'autre et par rapport audit endoscope.

En particulier, la présente invention vise à fournir un dispositif compatible non seulement avec son insertion par voie endoluminale (perorale ou transanale) mais également avec le placement *in* situ des instruments chirurgicaux dans la lumière du tube digestif.

Ainsi, notamment, la présente invention vise à fournir un dispositif qui offre une stabilité conformationnelle et mécanique suffisante pour le placement de ces instruments chirurgicaux dans la lumière du tube digestif.

Un autre but de l'invention est de fournir un dispositif qui permette le placement *in situ* d'instruments chirurgicaux dans le corps d'un patient au niveau d'autres cibles que celles comprises dans le tube digestif, et notamment de cibles situées au niveau de la cavité intrapéritonéale, telles que par exemple le pancréas.

Le dispositif selon l'invention permettrait ainsi la mise en oeuvre d'une grande variété de manipulations chirurgicales par voie endoluminale, augmentant le confort pour le patient.

Un autre but de la présente invention est de fournir un dispositif adaptable à une large gamme d'instruments chirurgicaux et d'endoscopes.

La présente invention est de fournir un dispositif adaptable à une large gamme de systèmes de vision autres que les endoscopes.

La présente invention vise également à fournir un dispositif qui soit facile d'utilisation et d'entretien, tout en garantissant une sécurité maximale.

### Résumé de l'invention

La présente invention se rapporte à une table ou support endoscopique selon la revendication 1, comprenant une bague d'assemblage à un endoscope et au moins deux bras opérateurs solidarisés à ladite bague d'assemblage et aptes à être traversés chacun par au moins un instrument chirurgical, lesdits bras opérateurs présentant chacun au moins un premier degré de liberté en rotation suivant lequel chacun des bras opérateurs est orientable indépendamment de l'autre.

Plus précisément, la présente invention se rapporte à un dispositif ou table endoscopique déployable comprenant :
- une bague d'assemblage à un endoscope, ladite bague correspondant à un élément tel qu'en position déployée dudit dispositif ou de ladite table, la hauteur dudit élément est plus petite que son diamètre et ledit élément présente une ouverture centrale telle que ladite bague est assemblable à l'extrémité distale d'un endoscope;
- au moins deux moyens de guidage ou bras opérateurs solidarisables à ladite bague d'assemblage, chacun desdits bras opérateurs étant apte à recevoir au moins un instrument chirurgical, et à être orienté indépendamment de l'autre bras selon au moins deux degrés de liberté en rotation dans un même premier plan d'orientation de façon telle que lorsqu'un instrument chirurgical est fixé audit bras, celui-ci est capable d'imposer audit instrument chirurgical un déplacement dans au moins deux dimensions de l'espace;
- des moyens d'orientation conçus pour orienter lesdits moyens de guidage ou bras opérateurs selon leurs degrés de liberté ;
- des moyens d'extension aptes à faire passer ledit dispositif ou ladite table endoscopique d'une position non déployée à un position déployée.

Le dispositif selon la présente invention présente donc la caractéristique d'être déployable, et plus précisément, sa bague d'assemblage est déployable.

Le premier plan d'orientation est défini par les deux axes principaux desdits bras opérateurs.

De préférence, chacun desdits bras présente un troisième degré de liberté en rotation tel que chacun desdits bras est orientable selon ce degré de liberté dans un deuxième plan d'orientation.

De préférence, le deuxième plan d'orientation des bras opérateurs est défini par l'axe principal dudit bras opérateur et un deuxième axe correspondant à l'axe essentiellement perpendiculaire au premier plan d'orientation.

De préférence, chacun desdits bras présente en outre un degré de liberté en translation correspondant au degré de liberté en translation le long de l'axe principal dudit bras.

De préférence, chacun desdits bras présente en outre un degré de liberté en rotation correspondant au degré de liberté en rotation le long de l'axe principal dudit bras.

Par exemple, les moyens d'extension du dispositif sont situés au moins en partie au niveau de la bague d'assemblage.

De façon particulièrement avantageuse, la bague d'assemblage comprend des moyens d'assemblage à distance à l'endoscope.

De préférence, les moyens d'assemblage à l'endoscope et les moyens d'extension du dispositif sont les mêmes.

De préférence, dans sa position déployée et hors fonctionnement, le dispositif selon l'invention présente un volume maximal *in* situ défini comme le volume pour lequel le dispositif présente *in situ* un maximum de stabilité conformationnelle et mécanique.

De préférence, dans sa position déployée et hors fonctionnement, ledit dispositif présente *in situ* un volume maximal qui est supérieur au volume minimal dudit dispositif en position non déployée d'au moins 10%, de préférence d'au moins 20%, de préférence d'au moins 30%, de préférence d'au moins 40%, de préférence d'au moins 50%, de préférence d'au moins 60%, de préférence d'au moins 70%, d'au moins 80%, d'au moins 90%, d'au moins 100%, d'au moins 200%, d'au moins 300%, d'au moins 500%, d'au moins 1000%, d'au moins 2000%.

De manière avantageuse, dans sa position déployée et lorsque le dispositif est hors fonctionnement, les bras opérateurs sont orientés de façon essentiellement perpendiculaire par rapport au plan de la bague et sont essentiellement parallèles l'un par rapport à l'autre.

De préférence, le dispositif selon l'invention est apte à être couplé à des moyens dits d'activation ou de contrôle manuels ou automatiques pour commander l'actionnement des moyens d'orientation et/ou l'actionnement des moyens d'extension.

Selon une première forme préférée d'exécution, la bague d'assemblage comprend une membrane flexible et un câble dit câble d'assemblage, ladite bague étant repliée sur elle-même en position non déployée du dispositif et déployée en position déployée dudit dispositif.

De préférence, les moyens d'extension comprennent ledit câble d'assemblage et un câble dit de repli et lesdits moyens d'extension sont actionnables par l'intermédiaire de guides rigides.

Selon une deuxième forme préférée d'exécution, la bague d'assemblage à l'endoscope se présente sous la forme d'une structure rigide avec un axe principal.

De préférence, les moyens d'extension de la table correspondent à un dispositif de pivotement qui est apte à faire pivoter ladite bague autour de son axe principal d'un certain angle.

Dans la première forme et la deuxième forme préférées d'exécution, de préférence, les moyens d'orientation comprennent un ensemble de câbles situés au niveau des bras opérateurs et actionnables en mouvement.

Selon une troisième forme d'exécution ne faisant pas partie de l'invention, la bague d'assemblage à l'endoscope comprend une membrane gonflable, la bague étant non gonflée en position non déployée du dispositif et gonflée en position déployée du dispositif.

De préférence, dans cette forme d'exécution, les moyens d'orientation des bras opérateurs se présentent sous la forme d'un circuit de circulation de fluide comprenant au moins des compartiments situés au niveau de la bague et susceptibles d'être remplis par un fluide selon un taux de remplissage ajustable.

De préférence, les moyens d'orientation des bras opérateurs se présentent sous la forme d'un circuit de circulation de fluide comprenant au moins des compartiments situés au niveau des bras opérateurs et susceptibles d'être remplis par un fluide selon un taux de remplissage ajustable.

Avantageusement, les moyens d'orientation des bras opérateurs se présente sous la forme d'un circuit de circulation de fluide comprenant des compartiments situés au niveau des bras opérateurs et au niveau de la bague d'assemblage, lesdits compartiments étant susceptibles d'être remplis par un fluide selon un taux de remplissage ajustable.

De préférence selon l'invention, les deux bras opérateurs sont souples.

De préférence selon l'invention, les deux bras opérateurs sont disposés en vis-à-vis l'un de l'autre par rapport au diamètre de la bague d'assemblage.

De préférence, les bras opérateurs sont pourvus de marqueurs optiques.

De préférence, au moins certains des éléments du dispositif sont à usage unique.

De manière alternative, le dispositif est nettoyable et est capable d'être réutilisé au moins une fois après nettoyage.

La présente description se rapporte également à un kit endoscopique comprenant un dispositif ou une table endoscopique selon l'invention et des moyens d'activation automatique ou des moyens d'activation manuelle des moyens d'orientation des bras opérateurs et/ou des moyens d'extension de la table.

La présente invention se rapporte également à un kit endoscopique comprenant un dispositif ou l'invention des revendications précédentes et un endoscope et/ou au moins un instrument chirurgical.

Un procédé pour pratiquer un acte chirurgical au niveau d'un site particulier dans le corps d'un patient est décrit, ledit procédé comprenant les étapes suivantes :
- on assemble les différents éléments du dispositif selon l'une des revendications précédentes,
- on introduit ledit dispositif en position non déployée par voie endoluminale dans le corps du patient jusqu'au site particulier à traiter,
- à l'aide des moyens d'extension on déploie *in situ* le dispositif,
- on assemble *in situ* audit dispositif un endoscope au niveau de l'extrémité distale dudit endoscope, en réglant la hauteur au niveau de l'extrémité distale dudit endoscope à laquelle l'assemblage est réalisé;
- on fixe sur un ou plusieurs des moyens de guidage ou bras opérateurs un ou plusieurs instruments chirurgicaux,
- on procède alors au traitement chirurgical en tant que tel en commandant via les moyens d'orientation des bras opérateurs l'orientation *in situ* des bras opérateurs dans l'espace et donc le déplacement des instruments dans l'espace, sous le contrôle du dispositif d'activation ;
- si nécessaire, en cours de procédure on fait varier l'orientation de l'endoscope par rapport au site à traiter et on fait varier les instruments chirurgicaux sur les bras opérateurs du dispositif endoscopique,
- une fois le traitement terminé, on enlève les instruments chirurgicaux du dispositif endoscopique, on désassemble *in situ* ledit dispositif endoscopique et l'endoscope, sous le contrôle des moyens d'extension, on commande le passage du dispositif endoscopique *in situ* d'une position déployée à une position non déployée, et on retire le dispositif endoscopique du corps du patient.

A la fin de la procédure, de préférence, on désassemble le dispositif endoscopique et on le jette ou on le nettoie en vue de son réemploi.

### Définitions :

On notera que dans la présente invention les termes « positionnement » et « orientation » sont liés l'un à l'autre.

On doit donc comprendre que dans la présente invention une position et/ou une orientation des bras opérateurs correspond à un jeu précis de coordonnées dans l'espace.

Ainsi que l'on pourra le comprendre aussi dans la présente description, la table endoscopique selon l'invention permet de positionner et/ou orienter un endoscope et des instruments chirurgicaux à une certaine hauteur dans le tube digestif d'un patient mais également une fois cette hauteur réglée d'affiner précisément la position et/ou l'orientation des bras opérateurs et des instruments chirurgicaux.

La notion de « degré de liberté » d'un système telle qu'utilisée dans la présente invention correspond à celle communément utilisée par l'homme de l'art en.mécanique et désigne a *priori* aussi bien un degré de liberté en rotation qu'un degré de liberté en translation.

La notion de « position d'assemblage » de la table renvoie à une conformation particulière de la table dans laquelle elle peut être assemblée à un endoscope. Cette position d'assemblage de la table correspond elle-même à une certaine conformation de la bague d'assemblage de la table.

Le dispositif ou la table endoscopique selon l'invention, qui comprend essentiellement la bague d'assemblage plus des bras opérateurs, est « déployable » c'est-à-dire qu'elle a la capacité à être déployée.

Dans la suite de la description, les termes « bras opérateurs » et « moyens de guidage » sont équivalents. L'expression « moyens de guidage » renvoie à la fonction des bras opérateurs qui est de guider ou encore d'imposer les mouvements dans l'espace des instruments chirurgicaux qui sont fixés sur ces bras par rapport au site du corps humain à traiter.

Les bras opérateurs servent donc à la fois de support et de guide aux instruments chirurgicaux. Chaque bras opérateur peut recevoir un ou plusieurs instruments chirurgicaux et est adaptable à une large gamme d'instruments chirurgicaux différant par leur forme, leur taille, leur poids, leur composition, ...

Dans la suite de la description, les termes « bague d'assemblage à un endoscope » ou « bague d'assemblage » ou « bague » sont équivalents et correspondent à un élément du dispositif endoscopique dont l'une des caractéristiques est que, lorsque le dispositif endoscopique est déployé, la hauteur dudit élément est inférieure à son diamètre.

On entend par « déploiement » du dispositif ou de la table endoscopique selon l'invention, l'action d'étalement dudit dispositif ou de ladite table. Cet étalement est tel que le volume dans laquelle la table peut être circonscrite, et qui est minimal avant déploiement, subit in situ une augmentation d'au moins 10%, de préférence d'au moins 20%, de préférence d'au moins 30%, de préférence d'au moins 40%, de préférence d'au moins 50%, de préférence d'au moins 60%, de préférence d'au moins 70%, d'au moins 80%, d'au moins 90%, d'au moins 100%, d'au moins 200%, d'au moins 300%, d'au moins 500%, d'au moins 1000%, d'au moins 2000%.

Dans la demande de brevet prioritaire EP 1607036, les termes « déployé » et « déplié » ont été utilisés l'un pour l'autre. Par souci de rigueur, on considérera néanmoins dans la présente invention que le terme « déployé » englobe le terme « déplié », qui fait référence à un étalement du dispositif par effet mécanique, et le terme « gonflé », qui fait référence à un étalement du dispositif par effet hydraulique et/ou pneumatique.

Le terme « déployé » est plus approprié car il est plus proche de la caractéristique réelle du dispositif selon l'invention. La présente description utilisera de ce fait le terme « déployé ».

Dans la demande de brevet prioritaire EP 1607036, les termes « non déployé » et « replié » ont été utilisés l'un pour l'autre. Par souci de rigueur, on considérera néanmoins dans la présente invention que le terme « non déployé » englobe le terme « replié », qui fait référence à un non étalement du dispositif par effet mécanique, et le terme « non gonflé », qui fait référence à un non étalement du dispositif par effet hydraulique et/ou pneumatique.

Le terme « non déployé » est plus approprié car il est plus proche de la caractéristique réelle du dispositif selon l'invention. La présente description utilisera de ce fait le terme « non déployé ».

On définit pour la table selon l'invention une position non déployée pour laquelle le volume dans lequel sont circonscrits les contours de la table est minimal.

Ce volume minimal est calculé en vue de respecter certaines contraintes d'utilisation. Plus précisément, ce volume minimal est calculé pour permettre l'insertion de la table dans le corps du patient par voie endoluminale, par exemple par l'oesophage.

On définit pour cette même table une position déployée pour laquelle le volume dans lequel sont circonscrits les contours de la table représente au moins 110%, de préférence au moins 120%, de préférence au moins 130%, de préférence au moins 140%, de préférence au moins 150%, de préférence au moins 160%, de préférence au moins 170%, au moins 180%, au moins 190%, au moins 200%, au moins 300%, au moins 400%, au moins 500%, au moins 1100%, au moins 2100% du volume minimal de la table en position non déployée. Ce volume maximal correspond au volume de la table lorsque celle-ci est déployée in situ. En position déployée, la table selon l'invention présente une stabilité conformationnelle et mécanique telle que les bras opérateurs peuvent *in situ* guider le déplacement dans l'espace des instruments chirurgicaux par rapport au site à traiter en toute sécurité. Cette stabilité du dispositif est telle qu'en pratique les bras opérateurs se substituent en quelque sorte aux mains du chirurgien.

Ce volume de la table en position déployée doit être cependant compatible avec l'utilisation qui est faite de la table et notamment il doit être adapté à la taille et à la conformation du site à traiter dans le corps. Ce site peut par exemple être l'estomac, mais d'autres sites dans le corps du patient peuvent encore être prévus et l'homme de l'art sera facilement adapté l'invention en fonction de la nature de ces sites.

Le passage de la table d'une position non déployée à une position déployée, et réciproquement le passage de la table d'une position déployée à une position non déployée est réalisé par des « moyens d'extension », eux-mêmes sous le contrôle de moyens d'activation qui peuvent être manuels ou automatiques.

On notera que la notion de « moyens d'extension » telle qu'utilisée dans la présente description est équivalente à la notion de « dispositif de repli/déploiement » telle qu'elle apparaissait dans la demande de brevet prioritaire EP 1607036.

On doit donc comprendre que le déploiement de la table selon l'invention a un caractère réversible.

Les moyens d'extension du dispositif peuvent être de différentes natures, ils peuvent notamment être de type hydraulique, pneumatique ou mécanique.

On notera d'ailleurs à ce sujet que la notion de « moyens d'activation » telle qu'utilisée dans la présente description est équivalente à la notion de « dispositif d'activation » telle qu'elle apparaissait dans la demande de brevet prioritaire EP 1607036.

La table est apte à être assemblée à un endoscope uniquement lorsqu'elle est en position déployée.

Dans cette position déployée de la table, l'ouverture centrale de la bague, encore appelée orifice ou trou endoscopique, a une conformation et une taille compatible avec l'assemblage du dispositif à l'extrémité distale d'un endoscope.

Cet assemblage a la particularité de pouvoir être réalisé à distance. De ce fait, l'assemblage de façon originale peut se faire *in situ.*

L'assemblage à l'endoscope est réalisé par des moyens d'assemblage à distance faisant partie de la bague.

La notion d'assemblage « à distance » traduit la capacité particulière de la bague, et donc de la table selon l'invention, à pouvoir être assemblée *in situ* c'est-à-dire dans le corps du patient, et plus précisément une fois que la table selon l'invention a atteint le site à traiter. Cette caractéristique technique distingue la table selon la présente invention de nombreux autres dispositifs endoscopiques qui ont été décrits dans l'état de la technique, tels que par exemple celui décrit dans le document US-B1-6,352,503, pour lesquels l'assemblage à l'endoscope doit nécessairement être réalisé à l'extérieur du corps du patient.

Ces moyens d'assemblage peuvent être soit des éléments mécaniques spécifiques, tels que par exemple des clips, mais ils peuvent être réalisés de manière indirecte par l'action de certains éléments de la bague remplissant d'autres fonctions. C'est ainsi que dans la forme gonflable de la table, les moyens d'assemblage peuvent être obtenus par la pression exercée autour de l'orifice ou trou endoscopique par le fluide circulant dans la bague, avant l'introduction du dispositif dans le corps du patient.

Ces moyens d'assemblage, et plus généralement la conformation de la bague en position déployée, sont conçus de façon à optimiser la surface de contact entre la bague et l'endoscope, tout en maintenant un maximum de flexibilité au niveau de l'endoscope.

En outre, la table endoscopique selon l'invention, et plus particulièrement les moyens d'assemblage, sont tels que l'assemblage peut se faire avec toutes sortes d'endoscopes disponibles dans le commerce, aussi bien à vision axiale qu'à vision latérale. La table endoscopique est adaptable à une large gamme d'endoscopes différant par leur forme, leur taille, leur poids, leur composition, ...

De façon plus générale encore, la table endoscopique est adaptable à une large gamme de systèmes de vision différant par leur forme, leur taille, leur poids, leur composition, ...

On notera également que la table endoscopique selon l'invention, et plus notamment les moyens d'assemblage, l'ouverture centrale de la bague, et les dimensions de la bague sont tels que l'assemblage de la table à un endoscope peut se faire à différentes hauteurs le long de l'extrémité distale de cet endoscope, sans pour autant entraver la flexibilité de l'endoscope.

On entend par « extrémité distale d'un endoscope » une zone de l'endoscope comprenant l'extrémité béquillable de cet endoscope, c'est-à-dire actionnable par des moyens de commande tels que des manettes, et une zone comprise entre environ 5 cm et environ 10 cm en amont.

Les moyens d'assemblage du dispositif à un endoscope et les moyens d'extension du dispositif peuvent être identiques.

Selon l'invention, les bras opérateurs du dispositif sont « solidarisables » à la bague d'assemblage.

En d'autres termes, dans le dispositif selon l'invention, les bras opérateurs sont solidarisés ou non solidarisés à la bague d'assemblage, mais ils ont en tout cas la capacité d'être solidarisés à ladite bague.

Les bras opérateurs sont suffisamment souples ou flexibles pour être orientables par des moyens d'orientation.

On notera que la notion de « moyens d'orientation » telle qu'utilisée dans la présente description est équivalente à la notion de « dispositif d'orientation » telle qu'elle apparaissait dans la demande de brevet prioritaire EP 1607036.

Tout comme les moyens d'extension, ces moyens d'orientation peuvent être de différentes natures, ils peuvent notamment être de type hydraulique, pneumatique ou mécanique.

Tout comme les moyens d'extension, les moyens d'orientation des bras opérateurs sont activables/actionnables par des moyens d'activation de type manuel ou automatique.

On notera que pour la table on définit une « position déployée hors fonctionnement de la table » comme une position déployée dans laquelle la table est apte à être assemblée à un endoscope et les moyens d'activation ne fonctionnent pas, c'est-à-dire les bras opérateurs ne bougent pas. Cette position déployée, correspond à une position de repos de la table dans laquelle les bras opérateurs sont orientés de façon essentiellement perpendiculaire par rapport au plan de la bague et sont essentiellement parallèles l'un par rapport à l'autre.

### Brève description des figures

Une première variante de la table ou du dispositif endoscopique selon une première forme préférée d'exécution est illustré par les figures 1a, 1b, 2a, 2b, 3a et 3b.

Les figures 1a et 1b représentent une vue générale d'un support ou d'une table endoscopique selon cette première forme préférée d'exécution de la présente invention en position déployée.

Les figures 2a-2b correspondent à deux vues différentes de cette même table endoscopique, avec un endoscope assemblé sur cette table.

Les figures 3a et 3b correspondent à deux vues différentes montrant en détail la bague d'assemblage à l'endoscope dans cette première forme préférée d'exécution d'une table selon l'invention.

Les figures 4 à 10 illustrent une autre variante de cette première forme d'exécution dans laquelle, par rapport à la variante des figures 1a-3b, la membrane de la bague d'assemblage a été remplacée par un câble. La figure 4 (vue générale) et la figure 5 (détail de la bague d'assemblage, vue du dessus) correspondent à la table sans endoscope et en position non déployée. Les figures 6 à 10 représentant différentes vues générales et de détail de cette même table en position déployée et assemblée à un endoscope.

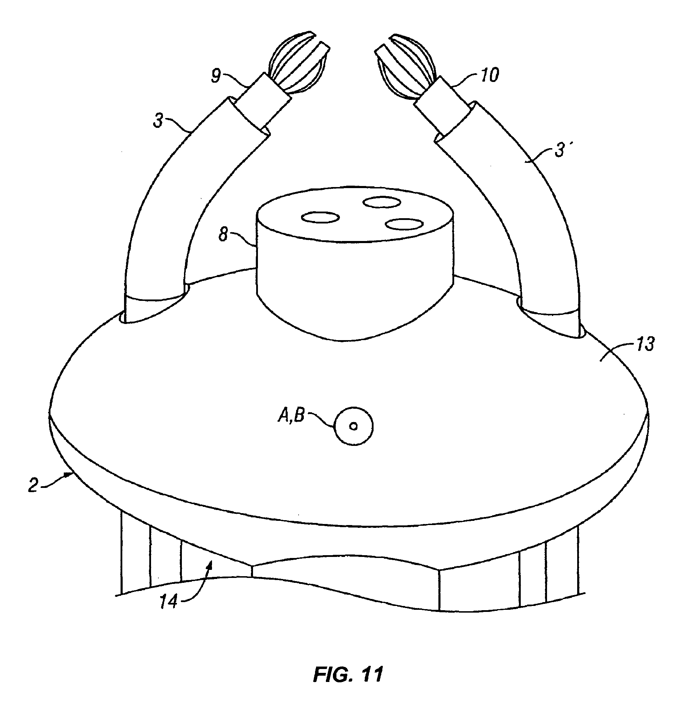

Une deuxième forme préférée d'exécution d'une table endoscopique selon la présente invention est illustrée par la figure 11.

### Description détaillée de l'invention

### Première forme préférée d'exécution- Première variante :

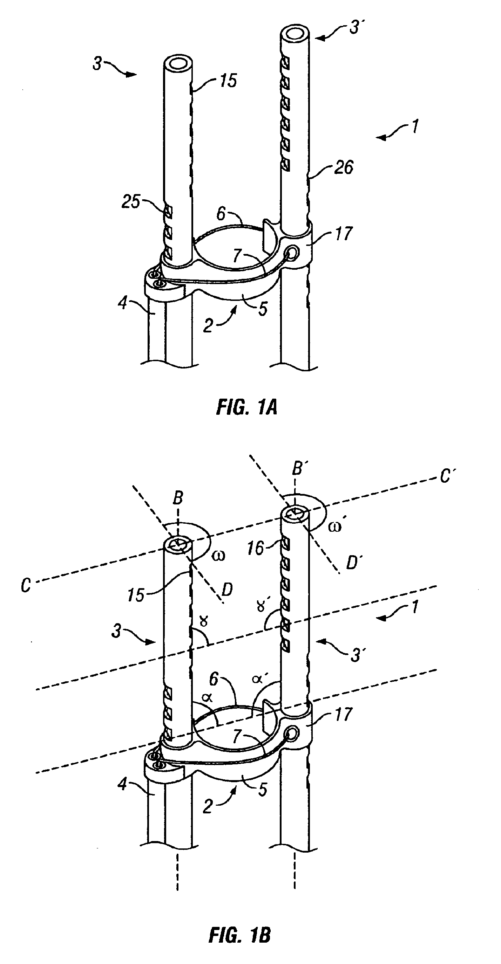

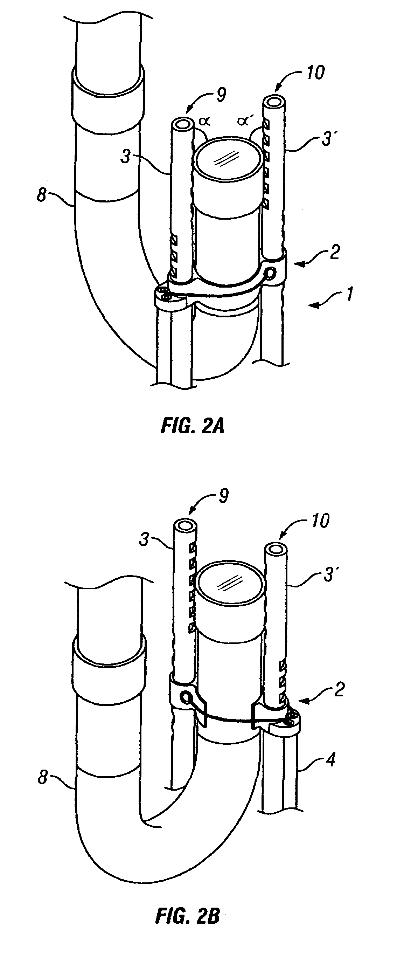

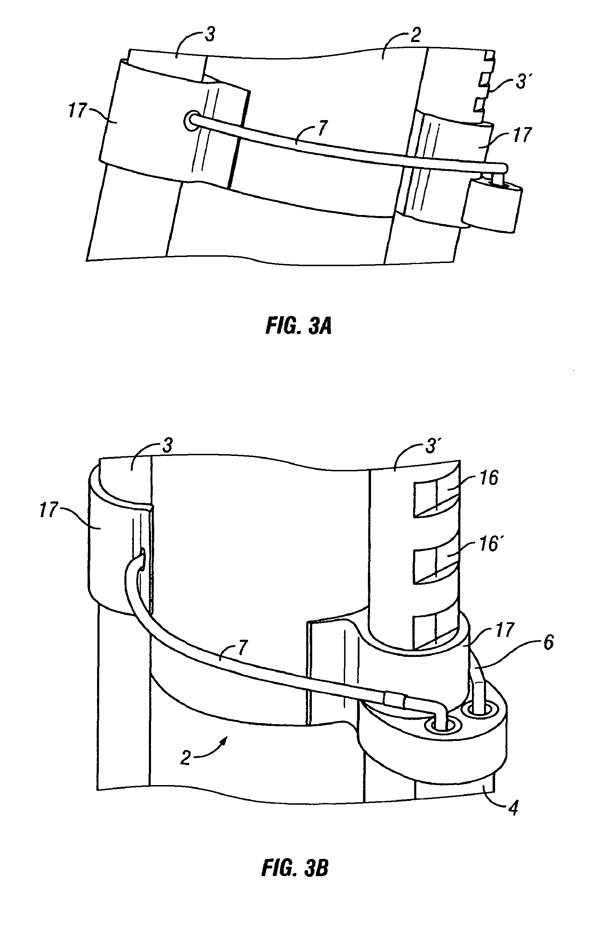

Un exemple d'une première forme préférée de la table opératoire selon l'invention est présenté aux figures 1a-1b, 2a-2b, 3a-3b.

La table endoscopique 1 comprend une bague d'assemblage ou interface d'assemblage 2 adaptée pour l'assemblage à un endoscope, et deux bras opérateurs 3,3' solidarisés à ladite bague d'assemblage 2 par des moyens de solidarisation 17, de préférence rigides.

Les bras opérateurs 3,3' se présentent sous la forme de tubes flexibles et comportent au moins un lumen ou canal configuré de façon telle que l'on peut y faire passer des outils ou instruments chirurgicaux de différentes formes.

La table endoscopique 1 présente au moins deux positions, une position non déployée correspondant à un volume minimal de ladite table tel que ladite table puisse être introduite dans le corps d'un patient par les voies naturelles (dites endoluminales c'est-à-dire perorales ou transanales) et une position de déploiement correspondant à une position d'assemblage à l'endoscope, c'est-à-dire une position dans laquelle un endoscope peut être assemblé à la table. Cette position est celle que la table peut adopter une fois qu'elle a atteint sa cible, par exemple l'estomac.

Des moyens d'extension de la table 1 sont prévus à cet effet (voir ci-après).

En position d'assemblage de la table, la bague d'assemblage 2 est déployée, tandis qu'en position non déployée de la table cette bague 2 est repliée sur elle-même.

Dans cette première forme préférée d'exécution, la bague d'assemblage 2 comprend une membrane flexible 5 qui contribue avec au moins un câble dit « câble d'assemblage » 6 à la forme de la bague 2 en position d'assemblage de la table 1.

La bague d'assemblage 2 comprend également au moins un câble dit « câble de repli » 7 qui permet de replier la bague 2 lorsque la table 1 passe de la position d'assemblage (déployée) à la position non déployée.

Le câble de repli 7 et le câble d'assemblage 6 forment ensemble les moyens d'extension de la bague 2 et donc de la table 1. Ce dispositif (6,7) est actionnable par l'intermédiaire de guides rigides 4, métalliques ou autres, disposés de préférence au niveau des bras opérateurs 3 et 3', mais d'autres moyens équivalents peuvent également être prévus à cet effet.

L'actionnement en mouvement (la mise en mouvement) du câble de repli 7 et du câble d'assemblage 6 des moyens d'extension est commandé soit manuellement soit automatiquement avec des moyens de contrôle par l'intermédiaire desdits guides rigides 4.

En position de repos de la table 1, les deux bras 3 et 3' sont orientés essentiellement parallèlement l'un par rapport à l'autre et essentiellement perpendiculairement au plan de la bague d'assemblage 2.

En outre, les deux bras 3 et 3' sont disposés en vis-à-vis l'un de l'autre par rapport au diamètre de la bague 2.

Avantageusement, les bras opérateurs 3 et 3' sont pourvus de marqueurs optiques (non représentés) bien connus de l'homme de métier pour permettre une visualisation in situ du dispositif, et notamment des bras opérateurs.

Ces marqueurs optiques peuvent par exemple prendre la forme de bandelettes radio-opaques disposées régulièrement le long des bras opérateurs, par exemple tous les centimètres dans les 10 derniers centimètres des bras opérateurs.

Selon un aspect essentiel de l'invention, chacun des bras opérateurs 3 et 3' présente au moins deux centre de rotation (degrés de liberté), α, γ et α', γ' respectivement, qui correspondent à deux rotations dans un premier plan d'orientation défini par les deux axes principaux B et B' desdits bras opérateurs 3 et 3' respectivement. Chacun des bras opérateurs 3 et 3' est orientable (inclinable) dans ce premier plan d'un certain angle α et γ (bras 3) ou α'et γ' (bras 3'), de façon indépendante grâce à un dispositif d'orientation.

En d'autres termes, les bras 3 et 3' sont orientables indépendamment l'un de l'autre dans au moins deux dimensions de l'espace, grâce à ce dispositif d'orientation.

Dans cette première forme préférée d'exécution, le dispositif d'orientation des bras opérateurs 3,3' (encore appelé dispositif de positionnement) comprend un ensemble de câbles 15, 25 d'une part et 16, 26, d'autre part, ces câbles étant situés au niveau respectivement du bras opérateur 3 et du bras opérateur 3' (deux séries de câbles sur chacun des bras opérateurs 3 et 3' assurant des déplacements en sens opposé). Ces câbles sont actionnables en mouvement par des moyens de contrôle mécaniques ou automatiques, encore appelés moyens d'activation ou moyens d'actionnement.

De façon générale, l'orientation des bras opérateurs 3 et 3' est ajustée par les moyens d'orientation de manière à ce que lorsque la table endoscopique est assemblée à l'endoscope, les bras opérateurs ne gênent pas la visibilité conférée par l'endoscope.

Il est à noter que les moyens d'orientation est conçu pour orienter les bras 3 et 3' avec des valeurs d'angles α, γ, α', γ' telles qu'en conditions d'utilisation, quand les instruments chirurgicaux 9,10 sont fixés auxdits bras 3 et 3', lesdits instruments chirurgicaux 9,10 se fassent toujours face.

De façon particulièrement avantageuse, chacun des bras opérateurs 3 et 3' présente en outre un autre degré de liberté (troisième degré de liberté) qui correspond à une rotation dans un deuxième plan d'orientation défini d'une part par l'axe principal B ou B' dudit bras opérateur 3 ou 3' respectivement et d'autre part par un deuxième axe D ou D' respectivement correspondant à un axe perpendiculaire au premier plan d'orientation défini ci-dessus. (On notera comme représenté sur la fig.lb que CBD et C'B'D' forment chacun deux trièdres rectangles). Chacun des bras opérateurs 3 et 3' est orientable (inclinable) dans ce deuxième plan d'orientation d'un certain angle ω ou ω' respectivement de façon indépendante grâce au dispositif d'orientation.

A cet effet, les moyens d'orientation des bras opérateurs 3,3' comprend des câbles supplémentaires 35, 45, ... et 36, 46, ... (non représentés sur les figures) situés au niveau des bras opérateurs respectivement 3 et 3' et actionnables en mouvement par des moyens de contrôle mécaniques ou automatiques de façon à orienter les bras 3 et 3' spécifiquement dans le deuxième plan d'orientation défini par ces angles ω et ω'.

En d'autres termes, avantageusement, les bras 3 et 3' sont orientables indépendamment l'un de l'autre dans les trois dimensions de l'espace, grâce aux moyens d'orientation.

D'autres câbles peuvent encore être ajoutés de façon à accroître, de manière indépendante, la mobilité des instruments chirurgicaux 9 et 10.

On peut également prévoir d'autres degrés de liberté pour les bras opérateurs 3 et 3' qui permettraient de les orienter encore plus précisément dans l'espace. Ainsi, on peut prévoir que les bras 3 et 3' soient également orientables en translation le long de leurs axes principaux et de façon indépendante l'un de l'autre et les moyens d'orientation peuvent être adaptés à cet effet.

On peut également prévoir de pouvoir faire tourner les bras opérateurs sur eux-mêmes (autour de leurs axes principaux respectifs).

En pratique, lorsqu'il s'agit d'utiliser la table opératoire selon l'invention, le chirurgien peut procéder de la façon suivante, par exemple.

La table opératoire 1 en position non déployée est introduite par voie endoluminale, par exemple perorale, dans le tube digestif du patient. Une fois que la table opératoire a atteint la cible à traiter, par exemple l'estomac ou l'oesophage, le chirurgien commande par voie manuelle ou automatique via l'actionnement des moyens d'extension le déploiement de la table 1 : la bague d'assemblage 2 qui était non déployée se déploie, alors. Plus précisément, le câble d'assemblage 6 est actionné et forme avec la membrane flexible 5 la bague d'assemblage 2 avec son orifice ou trou 11. La table 1 est alors dans sa position de repos ou d'équilibre.

L'endoscope 8 est alors inséré dans l'orifice 11 de la bague d'assemblage 2 et des instruments chirurgicaux 9 et 10 sont insérés au niveau des bras opérateurs 3 et 3' respectivement.

Les moyens d'orientation sont alors à leur tour actionnés, par les moyens de contrôle manuel ou automatique de manière à mettre en mouvement dans au moins deux directions de l'espace (angles α, γ et α' , γ' respectivement) les bras opérateurs 3 et 3', voire dans les trois directions de l'espace (angles α,γ et α' , γ' respectivement d'une part, et ω et ω' respectivement d'autre part, avec éventuellement une translation des bras 3 et 3'). Le résultat est le positionnement ainsi de façon précise des instruments chirurgicaux 9 et 10 respectivement par rapport à la cible à traiter et par rapport à l'endoscope 8.

La table endoscopique peut en outre être conçue de manière à permettre une orientation des instruments chirurgicaux 9 et 10 en translation dans les bras opérateurs 3 et 3' et en rotation autour de leurs axes, sous l'actionnement des moyens de contrôle.

Une fois les instruments ainsi correctement positionnés, la procédure chirurgicale opératoire peut commencer. Au cours de la procédure, les moyens de contrôle peuvent actionner les moyens d'orientation en vue d'ajuster à tout moment la position en 3D des bras opérateurs 3,3' et donc des instruments chirurgicaux 9,10.

Un système de sécurité incluant des moyens d'alarme peut en outre être prévu de manière à pouvoir à tout moment en cas de problème interrompre la procédure et désassembler si besoin rapidement la table opératoire 1, l'endoscope et les instruments chirurgicaux.

Une fois la procédure chirurgicale terminée, l'endoscope 8 et les instruments chirurgicaux 9,10 sont ôtés de la table opératoire 1 et les moyens d'extension sont à nouveau actionnés pour replier ladite table 1. La table 1 ainsi non déployée peut alors être retirée du corps du patient.

Il est à noter que la composition et les dimensions de la table endoscopique 1 sont telles qu'elles sont compatibles avec son utilisation. En d'autres termes on pourra sélectionner les matériaux pour fabriquer les différents éléments de la table parmi les matériaux biocompatibles tels que le silicone, les polymères PTFE, PVC, PP, PS, PET, un acier inoxydable, le nitinol ou le titane.

On choisira aussi la taille de la bague d'assemblage 2 (épaisseur, hauteur h et périmètre) de façon à optimiser la surface de contact avec l'endoscope et obtenir ainsi notamment un assemblage stable et sécurisé.

De même, en ce qui concerne la fabrication de la table endoscopique selon l'invention, différents procédés peuvent être envisagés pour fabriquer les différents éléments tels que l'injection, l'extrusion, le marquage au laser ou l'usinage.

En outre, l'assemblage de ces différents éléments pourra être réalisé selon différentes techniques telles que le collage, le soudage tel que le soudage ultrason, ou l'injection de bi-composants.

Par ailleurs, il est à noter que si dans l'exemple décrit ci-dessus les moyens d'extension de la bague d'assemblage 2 et donc de la table 1 comprennent un ensemble de câbles reliés à des guides rigides 4, métalliques ou autres, d'autres dispositifs alternatifs ayant la même fonction peuvent aussi être envisagés par exemple un dispositif à glissière interne, à glissière externe, un dispositif d'enroulement autour d'un bras opérateur.

On notera que dans cette forme d'exécution, la membrane flexible 5 peut être remplacée par tout moyen équivalent tel que par exemple un ou plusieurs câbles, ainsi qu'illustré dans la seconde variante (voir ci-dessous).

On pourrait également envisager que la membrane 5 ne soit plus flexible mais rigide.

### Première forme préférée d'exécution- Seconde variante :

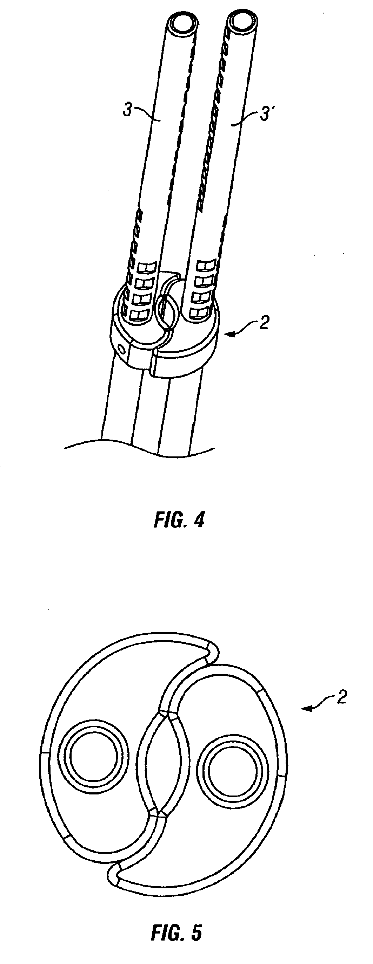

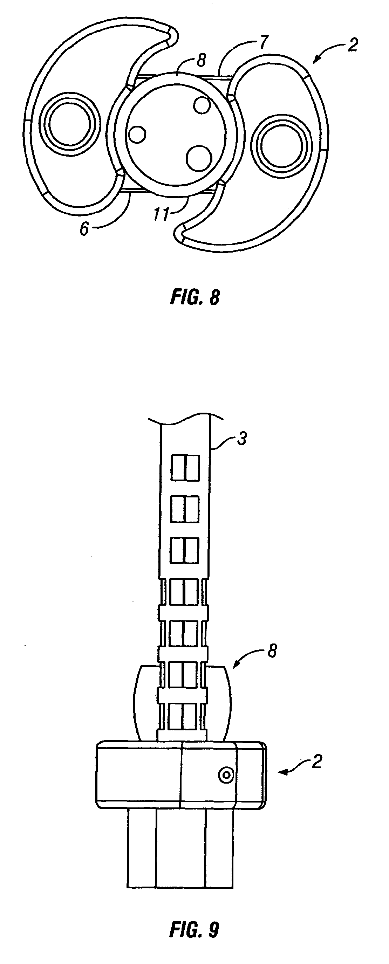

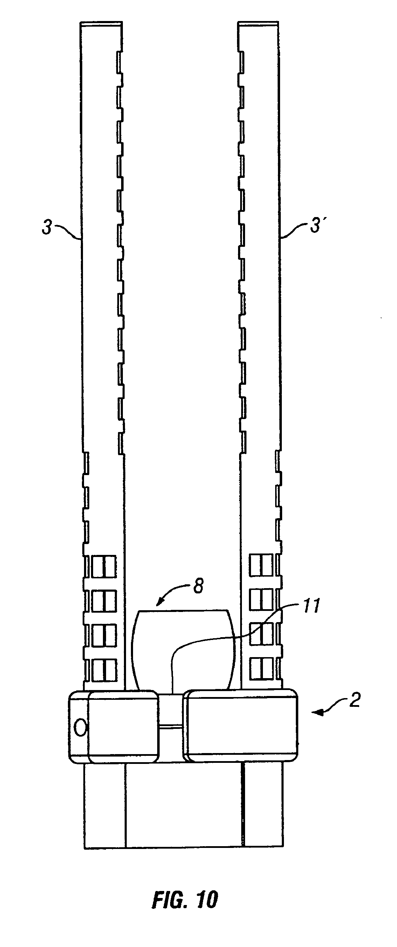

Les figures 4 à 10 illustrent une autre variante de cette première forme d'exécution dans laquelle, par rapport à la première variante des figures 1a-3b, la membrane de la bague d'assemblage a été remplacée par un câble.

La figure 4 (vue générale) et la figure 5 (détail de la bague d'assemblage) correspondent à la table sans endoscope et en position non déployée.

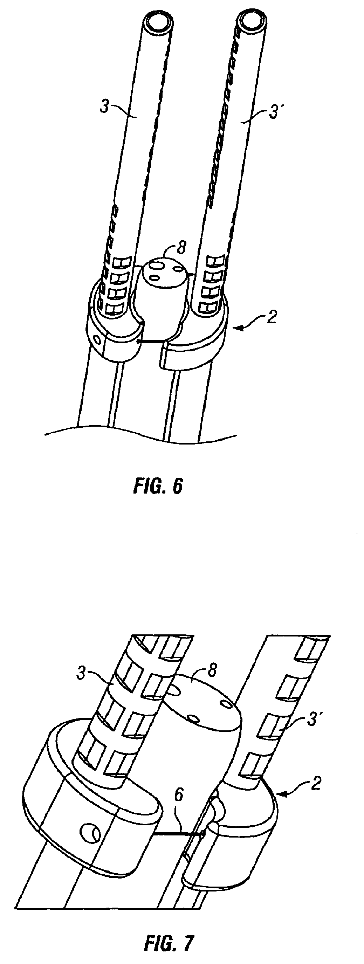

Les figures 6 à 10 représentent différentes vues générales et de détail de cette même table en position déployée et assemblée à un endoscope.

### Deuxième forme préférée d'exécution :

Une deuxième forme préférée d'exécution de la table endoscopique selon l'invention est représentée à la Fig.23.

Dans cette deuxième forme d'exécution, la bague ou interface d'assemblage 2 prend la forme d'une structure rigide capable de pivoter autour de l'axe A, de préférence d'un angle de pivotement ou de rotation β d'environ 90° de manière à être non déployée ou au contraire déployée. En position déployée, la bague ou interface d'assemblage 2 a son axe principal essentiellement perpendiculaire aux bras opérateurs 3,3'. Si on définit dans cette position une face nord 13 et une face sud 14 pour la bague 2, la position non déployée de la bague 2 correspond alors à une position de la bague dans laquelle la face nord 13 et la face sud 14 sont cette fois-ci situées dans des plans parallèles aux bras opérateurs 3,3'.

En d'autres termes, en pratique lorsqu'il s'agit d'introduire la table endoscopique 1 dans le corps d'un patient, par exemple dans l'oesophage, la table 1 est non déployée et donc la bague ou interface d'assemblage 2 expose sa face nord 13 et sa face sud 14 dans l'axe de l'oesophage.

Des moyens d'extension de la bague 2 et donc de la table endoscopique 1 à l'aide d'un câble relié à des guides rigides, métalliques ou autres, peuvent être prévus pour mettre en oeuvre un tel changement de conformation.

L'actionnement de ces moyens d'extension peut se faire, comme pour la première forme d'exécution par des moyens de commande ou contrôle manuels ou automatiques.

D'autres alternatives d'exécution de la table 1 peuvent encore être prévues telles que par exemple une table avec une demi-bague.

### Avantages de la table endoscopique selon l'invention :

Ainsi qu'illustré ci-dessus, grâce aux moyens d'orientation des bras opérateurs 3,3', il est possible avec la table endoscopique selon l'invention d'obtenir un positionnement précis des outils chirurgicaux dans le champ de vision de l'endoscope et selon un angle d'inclinaison variable de sorte que l'abord chirurgical s'en trouve facilité.

En outre, dans la mesure où les bras opérateurs 3,3' sont relativement flexibles, la table endoscopique selon l'invention peut être utilisée avec des outils chirurgicaux de formes très variées, y compris des outils chirurgicaux anguleux (non droits).

Un autre avantage de la table endoscopique selon l'invention est qu'elle n'est solidarisée ni aux outils chirurgicaux ni à l'endoscope. De par sa conception elle est donc capable de s'adapter à toutes sortes d'endoscopes et d'outils chirurgicaux.
Elle est en outre capable de s'adapter à toutes sortes de systèmes de vision, pas seulement des endoscopes.

De ce fait, même si la table endoscopique est avantageusement conçue pour être à usage unique, dans le cas où la table est conçue pour être réutilisée, son entretien s'en trouve également facilité tant au niveau simplicité qu'au niveau du coût et au niveau du temps.

En outre la table selon l'invention est telle que le chirurgien a la possibilité de changer in situ, en cours d'opération, de système de vision. Il peut ainsi adapter par exemple d'abord assembler la table à un endoscope à vision latérale, puis en cours d'opération, il peut changer cet endoscope et le remplacer par un endoscope à vision axiale, ou inversement.

De plus, la table est telle que le chirurgien peut facilement changer *in situ* la hauteur d'assemblage de l'endoscope sur la table, ce qui garantit à l'endoscope un maximum de flexibilité, sans entraver pour autant la visibilité du chirurgien.

Les applications médicales potentielles de l'instrument selon l'invention sont nombreuses et concernent notamment le traitement de pathologies telles que le reflux gastro-oesophagien, l'obésité morbide, la résection de tumeurs gastriques, la création d'anastomoses entre diverses structures digestives, et bien d'autres encore.

En conclusion, l'instrument selon la présente invention doit donc être compris comme un outil technique à la disposition du chirurgien pour faciliter la réalisation d'actes chirurgicaux tels que des sutures, des sections précises et des résections dans le cadre de l'endoscopie thérapeutique interventionnelle.

## Revendications

1. Dispositif ou table endoscopique (1) déployable d'une position non-déployée à une position déployée, comprenant :
- une bague d'assemblage (2) à un endoscope;
- au moins deux moyens de guidage ou bras opérateurs (3,3'), chacun desdits bras opérateurs (3,3') étant apte à recevoir au moins un instrument chirurgical;
- des moyens d'orientation conçus pour orienter lesdits moyens de guidage ou bras opérateurs indépendamment de l'autre moyens de guidage ou bras opérateur autour d'au moins deux centres de rotation (α,γ et α',γ' respectivement) dans un même premier plan d'orientation défini par les axes principaux (B, B') desdits moyens de guidage ou bras opérateurs, de façon telle que lorsqu'un instrument chirurgical est inséré dans ledit moyens de guidage ou bras opérateur, celui-ci est capable d'imposer audit instrument chirurgical un déplacement dans au moins deux dimensions de l'espace,
**caractérisé en ce que**:
- ladite bague correspond à un élément déployable tel qu'en position déployée dudit dispositif ou de ladite table, la hauteur dudit élément est plus petite que son diamètre et ledit élément présente une ouverture centrale telle que ladite bague est assemblable à l'extrémité distale d'un endoscope;
- les moyens de guidage ou bras opérateurs sont solidarisables à ladite bague d'assemblage (2); et le
- le dispositif comprend des moyens d'extension et d'assemblage de la bague (2) à l'endoscope, comprenant au moins un câble dit câble d'assemblage actionnable de manière à faire passer ledit dispositif ou ladite table endoscopique de la position non déployée à la position déployée par déploiement de la bague d'assemblage et former ladite ouverture centrale et, dans la position déployée, à assembler à distance la bague d'assemblage à l'endoscope par insertion de l'endoscope dans l'ouverture centrale.

2. Dispositif selon la revendication 1, **caractérisé en ce que** chacun desdits bras est orientable selon un degré de liberté (ω, ω' respectivement) en rotation dans un deuxième plan d'orientation.

3. Dispositif selon la revendication 2, **caractérisé en ce que** le deuxième plan d'orientation des bras opérateurs (3,3') est défini par l'axe principal (B ou B') dudit bras opérateur (3 ou 3') et un deuxième axe (D ou D') correspondant à l'axe essentiellement perpendiculaire au premier plan d'orientation.

4. Dispositif selon l'une quelconque des revendications précédentes, **caractérisé en ce que** chacun desdits bras présente en outre un degré de liberté en translation correspondant au degré de liberté en translation le long de l'axe principal dudit bras.

5. Dispositif selon l'une quelconque des revendications précédentes, **caractérisé en ce que** chacun desdits bras présente en outre un degré de liberté en rotation correspondant au degré de liberté en rotation le long de l'axe principal dudit bras.

6. Dispositif selon la revendication 1, **caractérisé en ce que** les moyens d'assemblage de la bague à l'endoscope et les moyens d'extension du dispositif sont les mêmes.

7. Dispositif selon l'une quelconque des revendications précédentes, **caractérisé en ce que** dans sa position déployée et lorsque le dispositif est hors fonctionnement, lesdits bras opérateurs sont orientés de façon essentiellement perpendiculaire par rapport au plan de la bague et sont essentiellement parallèles l'un par rapport à l'autre.

8. Dispositif selon l'une quelconque des revendications précédentes, **caractérisé en ce qu'**il est apte à être couplé à des moyens dits d'activation ou de contrôle manuels ou automatiques pour commander l'actionnement des moyens d'orientation et/ou l'actionnement des moyens d'extension et d'assemblage.

9. Dispositif selon l'une quelconque des revendications précédentes, **caractérisé en ce que** la bague d'assemblage (2) comprend une membrane flexible (5), ladite bague étant repliée sur elle-même en position non déployée du dispositif et déployée en position déployée dudit dispositif.

10. Dispositif selon la revendication 9, **caractérisé en ce que** les moyens d'extension et d'assemblage comprennent ledit câble d'assemblage (6) et un câble dit de repli (7) et lesdits moyens d'extension et d'assemblage sont actionnables par l'intermédiaire de guides rigides (4).

11. Dispositif selon l'une quelconque des revendications 1 à 8, **caractérisé en ce que** la bague d'assemblage à l'endoscope se présente sous la forme d'une structure rigide capable de pivoter autour d'un axe principal (A).

12. Dispositif selon la revendication 11, **caractérisé en ce que** les moyens d'extension de la table correspondent à un dispositif de pivotement qui est apte à faire pivoter ladite bague (2) autour de son axe principal (A) d'un certain angle (β).

13. Dispositif selon l'une quelconque des revendications précédentes, **caractérisé en ce que** les moyens d'orientation comprennent un ensemble de câbles (15,25; 16,26) situés au niveau des bras opérateurs (3, 3') et actionnables en mouvement.

14. Dispositif selon l'une quelconque des revendications précédentes, **caractérisé en ce que** les deux bras opérateurs (3,3') sont souples.

15. Dispositif selon l'une quelconque des revendications précédentes, **caractérisé en ce que** les deux bras opérateurs (3, 3') sont disposés en vis-à-vis l'un de l'autre par rapport au diamètre de la bague d'assemblage (2).

16. Dispositif selon l'une quelconque des revendications précédentes, **caractérisé en ce que** les bras opérateurs (3,3') sont pourvus de marqueurs optiques.

17. Kit endoscopique comprenant un dispositif ou une table endoscopique selon l'une quelconque des revendications 1 à 16 et un endoscope (8) et/ou au moins un instrument chirurgical (9 ou 10).

## Patentansprüche

1. Endoskopische Vorrichtung oder endoskopischer Tisch (1), die bzw. der aus einer nicht entfalteten Position in eine entfaltete Position entfaltbar ist, umfassend:
- einen Ring (2) zur Verbindung mit einem Endoskop,
- mindestens zwei Führungsmittel oder Bedienarme (3, 3'), wobei jeder der besagten Bedienarme (3,3') imstande ist, mindestens ein chirurgisches Instrument aufzunehmen,
- Orientierungsmittel, die hergestellt sind, um die besagten Führungsmittel oder Bedienarme unabhängig von dem anderen Führungsmittel oder Bedienarm um mindestens zwei Rotationszentren (jeweils α, γ und α', γ') herum in einer selben ersten Orientierungsebene zu orientieren, die von den Hauptachsen (B, B') der besagten Führungsmittel oder Bedienarme definiert ist, so dass, wenn ein chirurgisches Instrument in das besagte Führungsmittel oder den besagten Bedienarm eingesetzt ist, dieses imstande ist, dem besagten chirurgischen Instrument eine Verlagerung in mindestens zwei Dimensionen des Raums aufzuzwingen,
**dadurch gekennzeichnet, dass**:
- der Ring einem entfaltbaren Element entspricht, so dass in entfalteter Position der besagten Vorrichtung oder des besagten Tischs die Höhe des besagten Elements kleiner als sein Durchmesser ist und das Element eine zentrale Öffnung aufweist, so dass der besagten Ring mit dem distalen Ende eines Endoskops verbindbar ist,
- die Führungsmittel oder Bedienarme mit dem besagten Verbindungsring (2) fest verbindbar sind, und
- die Vorrichtung Erweiterungs- und Verbindungsmittel des Rings (2) mit dem Endoskop umfasst, die mindestens ein sogenanntes Verbindungskabel umfassen, das derart bedienbar ist, dass die besagte endoskopische Vorrichtung oder der besagten Tisch gewechselt wird von der nicht entfalteten nach der entfalteten Position durch Entfaltung des Verbindungsrings und das Bilden der zentralen Öffnung und, in der entfalteten Position, den Verbindungsring durch Einsetzen des Endoskops in die zentrale Öffnung mit dem Endoskop fernverbunden wird.

2. Vorrichtung nach Anspruch 1, **dadurch gekennzeichnet, dass** jeder der besagten Arme gemäß einem Rotationsfreiheitsgrad (jeweils ω, ω') in einer zweiten Orientierungsebene orientierbar ist.

3. Vorrichtung nach Anspruch 2, **dadurch gekennzeichnet, dass** die zweite Orientierungsebene der Bedienarme (3, 3') durch die Hauptachse (B oder B') des besagten Bedienarms (3 oder 3') und eine zweite Achse (D oder D'), die der zur ersten Orientierungsebene hauptsächlich senkrechten Achse entspricht, festgelegt ist.

4. Vorrichtung nach irgendeinem der vorhergehenden Ansprüche, **dadurch gekennzeichnet, dass** jeder der besagten Arme ferner einen Verschiebungsfreiheitsgrad aufweist, der dem Verschiebungsfreiheitsgrad entlang der Hauptachse des besagten Arms entspricht.

5. Vorrichtung nach irgendeinem der vorhergehenden Ansprüche, **dadurch gekennzeichnet, dass** jeder der Arme ferner einen Rotationsfreiheitsgrad aufweist, der dem Rotationsfreiheitsgrad entlang der Hauptachse des besagten Arms entspricht.

6. Vorrichtung nach Anspruch 1, **dadurch gekennzeichnet, dass** die Verbindungsmittel des Rings mit dem Endoskop und die Erweiterungsmittel der Vorrichtung dieselben sind.

7. Vorrichtung nach irgendeinem der vorhergehenden Ansprüche, **dadurch gekennzeichnet, dass** die besagten Bedienarme, in ihrer entfalteten Position und wenn die Vorrichtung außer Betrieb ist, im Wesentlichen senkrecht im Verhältnis zur Ebene des Rings orientiert sind und im Wesentlichen parallel zueinander sind.

8. Vorrichtung nach irgendeinem der vorhergehenden Ansprüche, **dadurch gekennzeichnet, dass** sie imstande ist an sogenannten manuelle oder automatische Aktivierungs- oder Kontrollmittel gekoppelt zu werden, um die Betätigung der Orientierungs- und/oder die Betätigung der Erweiterungs- und Verbindungsmittel zu steuern.

9. Vorrichtung nach irgendeinem der vorhergehenden Ansprüche, **dadurch gekennzeichnet, dass** der Verbindungsring (2) eine flexible Membran (5) umfasst, wobei der besagte Ring in nicht entfalteter Position der Vorrichtung zusammengefaltet ist und in entfalteter Position der besagten Vorrichtung entfaltet ist.

10. Vorrichtung nach Anspruch 9, **dadurch gekennzeichnet, dass** die Erweiterungs- und Verbindungsmittel das besagte Verbindungskabel (6) und ein sogenanntes Faltkabel (7) umfassen und die besagten Erweiterungs- und Verbindungsmittel mittels starrer Führungen (4) betätigbar sind.

11. Vorrichtung nach einem der Ansprüche 1 bis 8, **dadurch gekennzeichnet, dass** der Ring zur Verbindung mit dem Endoskop die Form einer starren Struktur hat, die um eine Hauptachse (A) schwenkbar ist.

12. Vorrichtung nach Anspruch 11, **dadurch gekennzeichnet, dass** die Erweiterungsmittel des Tischs einer Schwenkvorrichtung entsprechen, die imstande ist, den Ring (2) um seine Hauptachse (A) in einem bestimmten Winkel (β) zu schwenken.

13. Vorrichtung nach irgendeinem der vorhergehenden Ansprüche, **dadurch gekennzeichnet, dass** die Orientierungsmittel eine Gruppe von Kabeln (15, 25; 16, 26) umfassen, die sich im Bereich der Bedienarme (3, 3') befinden und zur Bewegung bedienbar sind.

14. Vorrichtung nach irgendeinem der vorhergehenden Ansprüche, **dadurch gekennzeichnet, dass** die zwei Bedienarme (3, 3') elastisch sind.

15. Vorrichtung nach irgendeinem der vorhergehenden Ansprüche, **dadurch gekennzeichnet, dass** die zwei Bedienarme (3, 3') im Verhältnis zum Durchmesser des Verbindungsrings (2) einander gegenüber angeordnet sind.

16. Vorrichtung nach irgendeinem der vorhergehenden Ansprüche, **dadurch gekennzeichnet, dass** die Bedienarme (3, 3') mit optischen Markern ausgestattet sind.

17. Endoskopieset, umfassend eine endoskopische Vorrichtung oder einen endoskopischen Tisch nach einem der Ansprüche 1 bis 16 und einen Endoskop (8) und/oder mindestens ein chirurgisches Instrument (9 oder 10).

## Claims

1. An endoscopy device or table (1) deployable from a non-deployed position to a deployed position, comprising:
- a connection ring (2) to an endoscope;
- at least two guiding means or operating arms (3, 3'), each of said operating arms (3, 3') being able to receive at least one surgical instrument;
- orientation means designed to orient said guiding means or operating arms independently of the other one guiding means or operating arm around at least two rotational centers (α, γ and α', γ', respectively) in a same first orientation plane defined by the main axes (B, B') of said guiding means or operating arms, such that when a surgical instrument is inserted into said guiding means or operating arm, the latter is capable of imposing a movement on said surgical instrument in at least two spatial dimensions,
**characterised in that**:
- said ring corresponds to a deployable element, such that in the deployed position of said device or said table, the height of said element is smaller than its diameter and said element has a central opening such that said ring can be connected to the distal end of an endoscope;
- the guiding means or operating arms can be secured to said connection ring (2); and
- the device comprises means for extension and for connection of the ring (2) to the endoscope, comprising at least one cable called connection cable that can be actuated so as to cause said endoscopy device or table to pass from the non-deployed position to the deployed position by deploying the connection ring and to form said central opening and, in the deployed position, to connect, at a distance, the connection ring to the endoscope by inserting the endoscope into the central opening.

2. The device according to claim 1, **characterised in that** each of said arms can be oriented with a rotational degree of freedom (ω, ω', respectively) in a second orientation plane.

3. The device according to claim 2, **characterised in that** the second orientation plane of the operating arms (3, 3') is defined by the main axis (B or B') of said operating arm (3 or 3') and a second axis (D or D') corresponding to the axis substantially perpendicular to the first orientation plane.

4. The device according to any one of the preceding claims, **characterised in that** each of said arms further has a translational degree of freedom corresponding to the translational degree of freedom along the main axis of said arm.

5. The device according to any one of the preceding claims, **characterised in that** each of said arms further has a rotational degree of freedom corresponding to the rotational degree of freedom along the main axis of said arm.

6. The device according to claim 1, **characterised in that** the means for connecting the ring to the endoscope and the extension means of the device are the same.

7. The device according to any one of the preceding claims, **characterised in that** in its deployed position and when the device is not in operation, said operating arms are oriented substantially perpendicularly relative to the plane of the ring and are substantially parallel to one another.

8. The device according to any one of the preceding claims, **characterised in that** it is able to be coupled to so-called activation or manual or automatic control means to control the actuation of the orientation means and/or the actuation of the extension and connection means.

9. The device according to any one of the preceding claims, **characterised in that** the connection ring (2) comprises a flexible membrane (5), said ring being folded on itself in the non-deployed position of the device and deployed in the deployed position of said device.

10. The device according to claim 9, **characterised in that** the extension and connection means comprise said connection cable (6) and a so-called fold-back cable (7) and said extension and connection means can be actuated via rigid guides (4).

11. The device according to any one of claims 1 to 8, **characterised in that** the connection ring to the endoscope is in the form of a rigid structure capable of pivoting around a main axis (A).

12. The device according to claim 11, **characterised in that** the extension means of the table correspond to a pivoting device that is able to pivot said ring (2) around its main axis (A) by a certain angle (β).

13. The device according to any one of the preceding claims, **characterised in that** the orientation means comprise a set of cables (15, 25; 16, 26) situated at the operating arms (3, 3') and able to be actuated in motion.

14. The device according to any one of the preceding claims, **characterised in that** the two operating arms (3, 3') are flexible.

15. The device according to any one of the preceding claims, **characterised in that** the two operating arms (3, 3') are positioned opposite each other relative to the diameter of the connection ring (2).

16. The device according to any one of the preceding claims, **characterised in that** the operating arms (3, 3') are provided with optical markers.

17. An endoscopic kit comprising an endoscopic device or table according to any one of claims 1 to 16 and an endoscope (8) and/or at least one surgical instrument (9 or 10).
